# EUROPEAN PATENT APPLICATION

(11) **EP 4 365 133 A1**
(43) Date of publication of application: **08.05.2024**
(21) Application number: 22205946.1
(22) Date of filing: 07.11.2022
(51) Int. Cl.: C01B 25/32, C09C 1/02, A61L 27/12, A61L 27/32, A61L 27/54, A61K 47/52, A61K 47/69, A61P 31/04

(54) **CRYSTALLINE HYBRID-LAYERED DICALCIUM PHOSPHATE, METHODS FOR PREPARATION AND USES THEREOF**

(71) Applicant: Gottfried Wilhelm Leibniz Universität Hannover, 30167 Hannover (DE)
(72) Inventor: Lu, Bingqiang, Shanghai (CN); Yang, Jinqin, Schweden (SE); Willhammar, Tom, Stockholm (SE); Hedin, Niklas, Stockholm (SE); Gebauer, Denis, Hannover (DE)
(74) Representative: Geling, Andrea

(57) **Abstract**

The present invention relates to a crystalline hybrid-layered dicalcium phosphate (HLCP), wherein the HLCP comprises alternating inorganic layers of dicalcium phosphate units and organic layers of molecules of at least one organic solvent or at least one organic active substance or a mixture of both; methods for preparation of HLCPs and uses of HLCPs.

## Description

The present invention relates to a crystalline hybrid-layered dicalcium phosphate (HLCP), wherein the HLCP comprises alternating inorganic layers of dicalcium phosphate units and organic layers of molecules of at least one organic solvent or at least one organic active substance or a mixture of both; methods for the preparation of HLCPs and uses of HLCPs.

### BACKGROUND

Calcium phosphates (CaPs) are the main inorganic constituents of calcified tissues in vertebrates, and probably the most important minerals to humans. Since 1769 when the existence in bones was first discovered by the Swedish chemist and metallurgist Johan Gottlieb Gahn¹, CaPs have been utilized in diverse biomedical applications^{2,3}. Synthetic CaPs including amorphous calcium phosphate (ACP)⁴, biphasic calcium phosphate⁵, tetra calcium phosphate⁶, octacalcium phosphate⁷, hydroxyapatite⁸, and so forth have especially thrived in orthopedics and dentistry as materials of choice for surgical fracture treatment and bone regeneration⁹. In today's markets, bone scaffolds, implants, and coating composites based on CaPs and their combinations with other metallic, polymeric and ceramic biomaterials are being pushed towards preclinical studies and clinical applications.

CaPs are regarded as typical inorganic compounds due to their elemental chemical constituents Ca, P, O, and H - or Ca²⁺, PO₄³⁻(and its derivatives), and H₂O considering the constituent ions and molecules. The great progress of the last decades in the field of organic-inorganic hybrid compounds, where organic molecules are combined with inorganics in different ways, has inspired the continually increasing exploration of these promising materials.

Compared to their purely organic or inorganic counterparts, hybrid materials often demonstrate enhanced properties, or even novel ones, and improved performances. Crystalline forms of hybrid CaP, i.e., where organic compounds are combined with CaP in the crystal lattice, seem to be promising to extend its application in biomedicine. Here, the presence of specific organic molecules alongside CaPs is frequently required, and these organic molecules may significantly change the crystalline hybrid CaP biomaterials' properties, as desired for their intended function.

However, developing crystalline hybrid CaP materials has been a great challenge and has rarely been reported to date.

One possible difficulty underlying crystalline hybrid CaP synthesis is that the formation of crystalline CaP phases often excludes the participation of organic molecules. In liquid solutions, e.g., water, organic solvents, or mixtures thereof, if a high amount of organic substance is present, amorphous CaP phases are typically yielded with the organic molecules adsorbed on them; if a low amount of organic substance is included, they fail in the competition against H₂O to insert into crystallographic lattice sites, leading to the formation of hydrated CaPs. Besides, organic molecules can often stabilize pure or hydrated CaPs, inhibiting their transformation into other phases, which, of course, includes crystalline hybrid CaPs.

Among the known CaPs, crystalline phases usually exhibit superior mechanical properties, which are highly advantageous in, e.g., bone tissue engineering, while amorphous CaPs possessing high surface areas are beneficial to cell colonization, vascularization, bone formation, and the diffusion of drugs, nutrients, and genes. The strategy to produce nanoscale crystalline CaPs can allow using them also for the latter purposes. Nanoscale crystalline CaPs benefit from both the dense matrix and high surface area and could show advantages in enhancing toughness and promoting osteoblast functions (such as adhesion, proliferation, and differentiation). However, the excessive dose risk caused by uncontrollable drug release from nano-particle surfaces and the danger of possible toxic responses of cells to nanoparticles remain an important issue.

The above-mentioned problems and drawbacks related to conventional CaPs and CaP-based biomaterials can potentially be solved by the invented, new family of hybrid layered dicalcium phosphates ("HLCPs"). These materials can also open up novel application potentials in orthopedics.

### SUMMARY OF THE INVENTION

Calcium phosphates (CaPs), the main mineral component of bones and teeth, constitute a class of compounds that vary in atomic structures and material properties for biomedical applications. In addition to calcium and phosphate ions, these typical inorganic minerals often include water molecules in their crystalline lattices and are hydrated phases. The present invention provides a new family of inorganic-organic hybrid layered calcium phosphate phases. Unlike conventional CaPs, organic molecules rather than water take part in building the crystalline structures and reside in the interlayer galleries of HLCP. The layered calcium hydrogen phosphate (calcium hydrogen phosphate is also named dicalcium phosphate) is structurally similar to dicalcium phosphate monohydrate (DCPM)¹⁷ but intercalated by layers of organic molecules instead of water molecules.

The HLCP phases of the present invention can be synthesized via the crystallization of, for example, amorphous dicalcium hydrogen phosphate (ACHP) in the respective organic solvents at ambient temperature. For example, for the preparation of HLCP-Me, HLCP-Fm, HLCP-DMF, and HLCP-DMSO, methanol (Me), formamide (Fm), dimethylformamide (DMF), and dimethyl sulfoxide (DMSO), respectively, are used as the incorporated organics. Moreover, these HLCP structures can transform from each other and from DCPM as well, in appropriate conditions.

The present invention is based on the idea that amorphous calcium hydrogen phosphate (ACHP), a special form of ACP, can serve as a precursor for various HLCPs.

Previous studies of the inventors showed that ACHP is featured with distinct microstructure and high metastability, by which it can even crystallize in solutions with a high content of up to 99.5% methanol, forming an already previously discovered, new crystalline phase, dicalcium phosphate monohydrate (DCPM).¹⁷ In the present invention, the inventors have further used pure organic solutions to induce the transformation of ACHP. The crystallization of ACHP is not prevented in this case, and moreover, organic molecules are not excluded upon building the crystalline structure. This yields a new family of hybrid layered dicalcium phosphate (HLCP), significantly expanding the structural richness of CaPs.

Thus, in a first aspect, the present invention relates to a hybrid-layered dicalcium phosphate (HLCP), wherein the HLCP is crystalline and comprises, or consists essentially of, or consists of alternating inorganic layers of dicalcium phosphate units and organic layers of molecules of at least one organic solvent or at least one organic active substance or a mixture of both.

In a second aspect, the present invention relates to the use of the inventive HLCP in bone tissue engineering or regenerative medicine, as or in bioceramics, as or in biocomposites, as or in bone scaffolds, as or in implants, as or in implant coatings, as or in CaP printable powders or liquids or as additives, and/or as an active substance carrier.

In a third aspect, the present invention relates to the use of an inventive HLCP comprising dimethyl sulfoxide as the at least one organic solvent molecule, as a medicament with analgesic, antiphlogistic, vasodilatory, and anti-edematous effects.

In a fourth aspect, the present invention relates to a method for preventing or reducing bacterial infections, preventing or reducing inflammations, treating bone or dental diseases, promoting the regeneration of bone or dental tissue, and/or preventing rejection of bone or dental implants comprising the step of implanting a material comprising an inventive HLCP carrying at least one organic active substance selected from the group consisting of antibiotics, antiphlogistics, drugs for the treatment of bone or dental diseases, drugs to promote regeneration of bone or dental tissue, and drugs to prevent rejection of bone or dental implants.

In a fifth aspect, the present invention relates to a method for treating pain, inflammations, hypertension, and/or edema comprising the step of implanting a material comprising an inventive HLCP, wherein the HLCP comprises dimethyl sulfoxide as the at least one organic solvent molecule.

In a sixth aspect, the present invention relates to a method for the manufacture of an inventive HLCP comprising the step of incubating a crystalline or precipitate calcium phosphate material with at least one organic solvent or at least one organic active substance or a mixture of both comprising 0 to 15 vol.% of water.

In a seventh aspect, the present invention relates to a method for the conversion of one inventive HLCP into another inventive HLCP, wherein an inventive HLCP is incubated with a different organic solvent.

This summary of the invention does not necessarily describe all features and/or all aspects of the present invention. Other embodiments will become apparent from a review of the ensuing detailed description.

### DETAILED DESCRIPTION

### Definitions

In the following the invention is described in more detail with reference to the Figures. The described specific embodiments of the invention, examples, or results are, however, intended for illustration only and should not be construed to limit the scope of the invention as indicated by the appended claims in any way.

It is to be understood that this invention is not limited to the particular methodology, protocols, and reagents described herein as these may vary. It is also to be understood that the terminology used herein is to describe particular embodiments only, and is not intended to limit the scope of the present invention which will be limited only by the appended claims. Unless defined otherwise, all technical and scientific terms used herein have the same meanings as commonly understood by one of ordinary skill in the art.

Each of the documents cited in this specification (including all patents, patent applications, scientific publications, manufacturer's specifications, instructions, etc.), whether supra or infra, is hereby incorporated by reference in its entirety. In the event of a conflict between the definitions or teachings of such incorporated references and definitions or teachings recited in the present specification, the text of the present specification takes precedence.

The term "comprising" or variations thereof such as "comprise(s)" according to the present invention (especially in the context of the claims) is to be construed as an open-ended term or non-exclusive inclusion, respectively (i.e., meaning "including, but not limited to,") unless otherwise noted.

The term "comprising" shall encompass and include the more restrictive terms "consisting essentially of" or "comprising substantially", and "consisting of".

In the case of chemical compounds or compositions, the terms "consisting essentially of" or "comprising substantially" mean that specific further components can be present, namely those not materially affecting the essential characteristics of the compound or composition, e.g., unavoidable impurities.

The terms "a", "an", and "the" as used herein in the context of describing the invention (especially in the context of the claims) should be read and understood to include at least one element or component, respectively, and are to be construed to cover both the singular and the plural, unless otherwise indicated herein or clearly contradicted by context.

In addition, unless expressly stated to the contrary, the term "or" refers to an inclusive "or" and not to an exclusive "or" (i.e., meaning "and/or").

All numeric values are herein assumed to be modified by the term "about", whether or not explicitly indicated. Recitation of ranges of values herein is merely intended to serve as a shorthand method of referring individually to each separate value falling within the range, unless otherwise indicated herein, and each separate value is incorporated into the specification as if it were individually recited herein.

The use of terms "for example", "e.g.", "such as", or variations thereof is intended merely to better illuminate the invention and does not pose a limitation on the scope of the invention unless otherwise claimed. These terms should be interpreted to mean "but not limited to" or "without limitation".

The term "dicalcium phosphate" as used herein, refers to the calcium phosphate with the formula CaHPO₄ and its hydrates. The "di" prefix in the common name arises because the formation of the HPO₄²⁻ anion involves the removal of two protons from phosphoric acid, H₃PO₄. It is also known as dibasic calcium phosphate or calcium (mono)hydrogen phosphate. The terms dicalcium phosphate and calcium hydrogen phosphate are used interchangeably here.

The term "crystal" or "crystalline" as used herein, refers to a solid material whose constituents (such as atoms, molecules, or ions) are arranged in a highly ordered microscopic structure, forming a crystal lattice that extends in all directions.

The term "amorphous" as used herein, refers to a solid that lacks the long-range order that is characteristic of a crystal. Amorphous materials may have some degree of short-range order at the atomic-length scale due to the nature of intermolecular chemical bonding.

The term "precipitate" as used herein, refers to the result of a process called precipitation meaning in chemistry the separation of a dissolved substance from a solution. Precipitation occurs when the solubility of a dissolved substance is exceeded due to changes in its environmental conditions, e.g., by addition of suitable substances (precipitants), changes in temperature and pressure, evaporation of the solvent, or changes in the polarity of the solvent. Precipitation takes place as a precipitate in the form of amorphous flakes or crystalline material.

The term "polar organic solvent" as used herein, refers to solvents that exhibit polar forces on solutes, due to high dipole moment, wide separation of charges, or tight association.

The term "dipole organic solvent" as used herein, refers to solvents that have an electric dipole moment as a measure of the separation of positive and negative electrical charges within a system, that is, a measure of the system's overall polarity.

The term "monoclinic unit cell" as used herein, refers to one of the seven crystal systems known in crystallography, i.e., the monoclinic crystal system. A crystal system is described by three vectors. In the monoclinic system, the crystal is described by vectors of unequal lengths. They form a rectangular prism with a parallelogram as its base. Hence two pairs of vectors are perpendicular (meet at right angles), while the third pair makes an angle other than 90°.

The term "space group" as used herein, has the usual meaning in crystallography, i.e., refers to the symmetry group of an object in space, usually in three dimensions. The elements of a space group (its symmetry operations) are the rigid transformations of an object that leave it unchanged. In three dimensions, space groups are classified into 219 distinct types, or 230 types if chiral copies are considered distinct.

The term "non-porous" as used herein, refers to a material having no void (i.e., "empty") spaces inside.

The term "antibiotics" as used herein, refers to a type of antimicrobial substances active against bacteria and widely used in the treatment and prevention of bacterial infections. They may either kill or inhibit the growth of bacteria.

The term "antiphlogistics" as used herein, refers to substances with an anti-inflammatory property used for treatment that reduces inflammation or swelling.

The term "analgesic" as used herein, refers to a group of drugs used to achieve relief from pain. Analgesic drugs are also called pain relievers or painkillers. Analgesics are used to induce cooperation with a medical procedure.

The term "vasodilatory" as used herein, refers to the effect of a group of drugs, "vasodilators", to widen blood vessels. the vasodilatory effect results from the relaxation of smooth muscle cells within the vessel walls, in particular in the large veins, large arteries, and smaller arterioles. When blood vessels dilate, the flow of blood is increased due to a decrease in vascular resistance and an increase in cardiac output. Therefore, dilation of arterial blood vessels (mainly the arterioles) decreases blood pressure.

The term "anti-edematous" as used herein, refers to the effect of a group of drugs to prevent or eliminate the accumulation of water in tissue (edema). For example, edema may be treated with drugs ("diuretics") that help the body expel excess fluid in the form of urine.

The term "bone tissue engineering" as used herein, refers to a technique that aims to induce new functional bone regeneration via the synergistic combination of biomaterials, cells, and factor therapy.

The term "regenerative medicine" as used herein, refers to any process of replacing, engineering, or regenerating human or animal cells, tissues or organs to restore or establish normal function. Damaged tissues and organs are stimulated by the body's own repair mechanisms to functionally heal previously irreparable tissues or organs.

The term "bioceramic" as used herein, refers to ceramic materials that are biocompatible. Bioceramics are an important subset of biomaterials and range in biocompatibility from bioceramics, which are inert in the body, to the other extreme of resorbable materials, which are eventually replaced by the body after they have assisted repair.

The term "biocomposite" as used herein, refers to a composite material formed by a matrix (e.g., a resin) and a reinforcement of bio (natural) fibers (e.g., fibers from crops such as cotton, flax, or hemp, recycled wood, waste paper, crop processing byproducts, or regenerated cellulose fibers). The matrix phase is usually formed by polymers derived from renewable and nonrenewable resources and protects the biofibers from environmental degradation and mechanical damage, holds the biofibers together, and transfers the loads on it.

The term "bone scaffolds" as used herein refers to three-dimensional biomaterial (i.e., a material that has been engineered to interact with biological systems for a medical purpose) structures used for bone defect reconstruction. Scaffolds play a crucial role in bone tissue engineering. Their purpose is to mimic the structure and function of the natural bone extracellular matrix (ECM), which can provide a three-dimensional (3D) environment to promote adhesion, proliferation, and differentiation and to have adequate physical properties for bone repair. An ideal scaffold should be biodegradable, biocompatible, bioactive, osteoconductive, and osteoinductive. Artificial bone scaffolds with biomaterials and additives, such as drugs, growth factors (GFs), and stem cells, have been useful for bone repair.

The term "implant", as used herein (e.g., in connection with bone or dental implants), refers to an artificial material intended for placement in a human (or animal) body. In particular, the term "implant", as used herein, refers to an artificial material implanted in the body to remain there permanently or at least for a longer period of time. The term "implant", as used herein, can be applied to the entire spectrum of medical devices intended for placement in a human (or animal) body. An example of an implant is a prosthesis.

The implant described herein is preferably implanted into the body of an individuum, e.g. into a human (or animal) body.

The term "coating" as used herein, refers to any layer covering a surface. In the present invention, the inventive HLCP can be used for coating or as a coating. It is preferred that the coating completely and/or uniformly covers the surface of the material to be coated. The coating can be achieved, e.g., by dip coating and/or spray coating. The coating is preferably a film. The coating may have any thickness, for example, the coating can form a film.

The term "printable powder" as used herein, refers to a powder suitable for 3D printing. There are two main powder printing processes: powder bed fusion and binder jetting. With powder bed fusion, powder 3D printers either sinter or melt powder particles with a laser into the desired object layer-by-layer, while a recoating blade adds more powder for each new layer. In place of a laser, binder jetting uses a print head that deposits a liquid bonding agent to the powder print bed. The liquid binds powder particles together to form each layer of the desired object. A fresh coat of powder is then added, and the process repeats layer by layer.

The term "active substance" as used herein, refers to any component that provides a biologically active or other direct effect in the diagnosis, cure, mitigation, treatment, or prevention of disease or to affect the structure or any function of the body of humans or animals. The similar term active pharmaceutical ingredient (also abbreviated as API) is also used in medicine, and some medication products may contain more than one active ingredient.

No language in this specification should be construed as indicating any non-claimed element as essential to the practice of the invention.

### Embodiments

The present invention is based on the unexpected finding that (almost) pure organic solvents can be used to induce the transformation of amorphous calcium hydrogen phosphate (ACHP) (and other calcium phosphate materials). Surprisingly, the crystallization of ACHP is not prevented, and moreover, the organic molecules of the solvents are not excluded upon building the crystalline structure.

Thus, in a first aspect, the present invention relates to a hybrid-layered dicalcium phosphate (HLCP), wherein the HLCP is crystalline and comprises, or consists essentially of, or consists of alternating inorganic layers of dicalcium phosphate units and organic layers of molecules of at least one organic solvent or at least one organic active substance or a mixture of both.

In one embodiment of the present invention, the molecules of the at least one organic solvent are molecules of a polar or dipole organic solvent.

In another embodiment of the present invention, the molecules of the at least one organic solvent are selected from the group consisting of methanol, formamide, dimethyl sulfoxide, dimethylformamide, glycerol, ethylene glycol, ethanol, and mixtures thereof.

Thus, the inventive HLCPs comprise, e.g., crystalline dicalcium phosphate methanol (CaHPO₄ • CH₃OH), crystalline dicalcium phosphate formamide (CaHPO₄ • HCONH₂), crystalline dicalcium phosphate dimethyl sulfoxide (CaHPO₄ • (CH₃)₂SO), crystalline dicalcium phosphate dimethylformamide (CaHPO₄ • HCON(CH₃)₂), crystalline dicalcium phosphate glycerol (CaHPO₄ • HO-CH₂-CH(OH)-CH₂-OH), crystalline dicalcium phosphate ethylene glycol (CaHPO₄ • HO-CH₂-CH₂-OH), and crystalline dicalcium phosphate ethanol (CaHPO₄ • C₂H₅OH). It should be noted that the above chemical formulas, e.g. CaHPO₄ • CH₃OH, are for abbreviation purposes only and do not necessarily mean a 1:1 stoichiometry.

In one embodiment of the present invention, the dicalcium phosphate methanol crystals have a monoclinic unit cell with space group P2₁/c and unit cell parameters a = 1.0153 nm, b = 0.6814 nm, c = 0.7725 nm, α = γ = 90°, β = 106.001; the dicalcium phosphate formamide crystals have a monoclinic unit cell with space group P2₁/c and unit cell parameters a = 1.0470 nm, b = 0.6784 nm, c = 0.7879 nm, α = γ = 90°, β = 105.578°; the dicalcium phosphate dimethyl sulfoxide crystals have a monoclinic unit cell with space group P2₁/c and unit cell parameters a = 1.12442 nm, b = 0.6759 nm, c = 0.7803 nm, α = γ = 90°, β = 106.065°; or the dicalcium phosphate dimethylformamide crystals have a monoclinic unit cell with space group P2₁/c and unit cell parameters a = 1.209 nm, b = 0.6774 nm, c = 0.782 nm, α = γ = 90°, β = 107.24°. The skilled artisan appreciates that a crystal structure is not substance-specific, i.e., a substance with a certain chemical composition can have different thermodynamically stable structures depending on external conditions. Thus, different crystal structures (also named modifications) of the above inventive crystalline HLCPs can exist.

In one embodiment of the present invention, the distance between the inorganic layers is 0.98 nm for methanol as an organic layer, 1.01 nm for formamide as an organic layer, 1.08 nm for dimethyl sulfoxide as an organic layer, and 1.15 nm for dimethylformamide as an organic layer.

In another embodiment of the present invention, the methanol, formamide, dimethyl sulfoxide, dimethylformamide, glycerol, ethylene glycol, and ethanol is held together by the inorganic layers in the dicalcium phosphate methanol, dicalcium phosphate formamide, dicalcium phosphate dimethyl sulfoxide, dicalcium phosphate dimethylformamide, dicalcium phosphate glycerol, dicalcium phosphate ethylene glycol, and dicalcium phosphate ethanol.

In yet another embodiment of the present invention, the methanol and formamide reside in the interlayer gallery (i.e., a crystalline layer of the organic molecules between two layers of dicalcium phosphate within the crystal structure) of dicalcium phosphate methanol and dicalcium phosphate formamide.

In one embodiment of the present invention, in the dicalcium phosphate methanol and in the dicalcium phosphate formamide the distance O^Me to O^HLCP and O^Fm to O^HLCP is between 0.25 and 0.32 nm, wherein the symbol ^ stands for a "coupling", i.e., to which part of the respective component of the HLCP the "O" belongs. For example, O^Me means the O of a methanol molecule and O^HLCP means an O of the phosphate group of a dicalcium phosphate unit. The above-mentioned distance is the distance between the different "O"s.

In another embodiment of the present invention, the HLCP is non-porous.

In yet another embodiment of the present invention, the at least one organic active substance is selected from the group consisting of antibiotics, antiphlogistics, drugs for the treatment of bone or dental diseases, drugs to promote regeneration of bone or dental tissue, and drugs to prevent rejection of bone or dental implants.

In yet another embodiment of the present invention, the at least one organic active substance is selected from the group consisting of hydrogen peroxide, triclosan, penicillin, povidone iodine, amoxicillin, clindamycin, doxycycline, gentamycin, chlorhexidine, rifamycin, vancomycin, LL-27, parathormone, BMP-2, BMP-7, TGF-beta, BMP-3, siRNA, romosozumab, and mixtures thereof.

In a second aspect, the present invention relates to the use of the inventive HLCP in bone tissue engineering or regenerative medicine, as or in bioceramics, as or in biocomposites, as or in bone scaffolds, as or in implants, as or in implant coatings, as or in CaP printable powders or liquids or as additives, and/or as an active substance carrier. As to the preferred embodiments of the inventive HLCP, it is referred to the first aspect of the present invention.

In one embodiment of the present invention, the implants are bone or dental implants or the implant coating is a bone or dental implant coating.

In a third aspect, the present invention relates to the use of an inventive HLCP comprising dimethyl sulfoxide as the at least one organic solvent molecule, as a medicament with analgesic, antiphlogistic, vasodilatory, and anti-edematous effects.

In a fourth aspect, the present invention relates to a method for preventing or reducing bacterial infections, preventing or reducing inflammations, treating bone or dental diseases, promoting the regeneration of bone or dental tissue, and/or preventing rejection of bone or dental implants comprising the step of implanting a material comprising an inventive HLCP carrying at least one organic active substance selected from the group consisting of antibiotics, antiphlogistics, drugs for the treatment of bone or dental diseases, drugs to promote regeneration of bone or dental tissue, and drugs to prevent rejection of bone or dental implants.

The fourth aspect may alternatively be formulated as follows: Inventive HLCP for use in preventing or reducing bacterial infections, preventing or reducing inflammations, treating bone or dental diseases, promoting the regeneration of bone or dental tissue, and/or preventing rejection of bone or dental implants, wherein the inventive HLCP carries at least one organic active substance selected from the group consisting of antibiotics, antiphlogistics, drugs for the treatment of bone or dental diseases, drugs to promote regeneration of bone or dental tissue, and drugs to prevent rejection of bone or dental implants.

For this purpose, the material comprising an inventive HLCP is implanted, specifically into the body of an individuum.

In a fifth aspect, the present invention relates to a method for treating pain, inflammations, hypertension, and/or edema comprising the step of implanting a material comprising an inventive HLCP, wherein the HLCP comprises dimethyl sulfoxide as the at least one organic solvent molecule.

The fifth aspect may alternatively be formulated as follows: Inventive HLCP for use treating pain, inflammations, hypertension, and/or edema, wherein the HLCP comprises dimethyl sulfoxide as the at least one organic solvent molecule.

For this purpose, the material comprising an inventive HLCP is implanted, specifically into the body of an individuum.

In a sixth aspect, the present invention relates to a method for the manufacture of an inventive HLCP, e.g. the inventive HLCP according to the first aspect, comprising the step of incubating a crystalline or precipitate calcium phosphate material with at least one organic solvent or at least one organic active substance or a mixture of both comprising 0 to 15 vol.% of water. For example, the water content may be 0, 0.1, 0.2, 0.3, 0.4, 0.5, 0.6, 0.7, 0.8, 0.9, 1, 2, 3, 4, 5, 6, 6.5, 7, 8, 9, 10, 11.2, 12, 13, 14, or 15 vol%.

In one embodiment of the present invention, the calcium phosphate material is selected from the group consisting of amorphous dicalcium phosphate containing 0-2 moles (e.g., 0, 0.4, 1, 1.2, 1.8, 2) of water per mole of dicalcium phosphate, crystalline dicalcium phosphate monohydrate, and dicalcium phosphate dihydrate. The at least one organic solvent is a polar or dipole organic solvent that, for example, can be selected from the group consisting of methanol, formamide, dimethyl sulfoxide, dimethylformamide, glycerol, ethylene glycol, and ethanol.

In another embodiment of the present invention, the above-mentioned incubation is carried out at 25 ± 5 °C (e.g., 20, 21, 22, 23, 24, 25, 26, 27, 28, 29, or 30 °C) for about 24 hours (e.g., 8, 10, 12, 15, 18, 20, 22, or 24 h), preferably with shaking at about 90 rpm (e.g., 50, 60, 70, 80, 90, 100, 110, 120, or 130 rpm).

In a seventh aspect, the present invention relates to a method for the conversion of one inventive HLCP into another inventive HLCP, wherein an inventive HLCP is incubated with a different organic solvent.

In one embodiment of the present invention, the afore-mentioned incubation is carried out at 25 ± 5 °C (e.g., 20, 21, 22, 23, 24, 25, 26, 27, 28, 29, or 30 °C) for about 24 hours (e.g., 8, 10, 12, 15, 18, 20, 22, or 24 h), preferably with shaking at about 90 rpm (e.g., 50, 60, 70, 80, 90, 100, 110, 120, or 130 rpm).

All uses of the HLCPs described above may be *in vitro* or *in vivo* uses. All methods described above may be *in vitro* or *in vivo* methods.

The invention is summarized as follows:
1. A hybrid-layered dicalcium phosphate (HLCP), wherein the HLCP is crystalline and comprises, or consists essentially of, or consists of alternating inorganic layers of dicalcium phosphate units and organic layers of molecules of at least one organic solvent or at least one organic active substance or a mixture of both.
2. The HLCP according to item 1, wherein the molecules of the at least one organic solvent are molecules of a polar or dipole organic solvent.
3. The HLCP according to items 1or 2, wherein the molecules of the at least one organic solvent are selected from the group consisting of methanol, formamide, dimethyl sulfoxide, dimethylformamide, glycerol, ethylene glycol, ethanol, and mixtures thereof.
4. The HLCP according to any one of items 1 to 3, wherein the HLCP is crystalline dicalcium phosphate methanol (CaHPO₄ • CH₃OH), crystalline dicalcium phosphate formamide (CaHPO₄ • HCONH₂), crystalline dicalcium phosphate dimethyl sulfoxide (Ca-HPO₄ • (CH₃)₂SO), crystalline dicalcium phosphate dimethylformamide (CaHPO₄ • HCON(CH₃)₂), crystalline dicalcium phosphate glycerol (CaHPO₄ • HO-CH₂-CH(OH)-CH₂-OH), crystalline dicalcium phosphate ethylene glycol (CaHPO₄ • HO-CH₂-CH₂-OH), or crystalline dicalcium phosphate ethanol (CaHPO₄ • C₂H₅OH).
5. The HLCP according to item 4, wherein the dicalcium phosphate methanol crystals have a monoclinic unit cell with space group P2₁/c and unit cell parameters a = 1.0153 nm, b = 0.6814 nm, c = 0.7725 nm, α = γ = 90°, β = 106.001; the dicalcium phosphate formamide crystals have a monoclinic unit cell with space group P2₁/c and unit cell parameters a = 1.0470 nm, b = 0.6784 nm, c = 0.7879 nm, α = γ = 90°, β = 105.578°; the dicalcium phosphate dimethyl sulfoxide crystals have a monoclinic unit cell with space group P2₁/c and unit cell parameters a = 1.12442 nm, b = 0.6759 nm, c = 0.7803 nm, α = γ = 90°, β = 106.065°; or the dicalcium phosphate dimethylformamide crystals have a monoclinic unit cell with space group P2₁/c and unit cell parameters a = 1.209 nm, b = 0.6774 nm, c = 0.782 nm, α = γ = 90°, β = 107.24°.
6. The HLCP according to any one of items 3 to 5, wherein the distance between the inorganic layers is 0.98 nm for methanol as an organic layer, 1.01 nm for formamide as an organic layer, 1.08 nm for dimethyl sulfoxide as an organic layer, and 1.15 nm for dimethylformamide as an organic layer.
7. The HLCP according to any one of items 3 to 6, wherein the methanol, formamide, dimethyl sulfoxide, dimethylformamide, glycerol, ethylene glycol, and ethanol is held together by the inorganic layers in the dicalcium phosphate methanol, dicalcium phosphate formamide, dicalcium phosphate dimethyl sulfoxide, dicalcium phosphate dimethylformamide, dicalcium phosphate glycerol, dicalcium phosphate ethylene glycol, and dicalcium phosphate ethanol.
8. The HLCP according to any one of items 3 to 7, wherein the methanol and formamide reside in the interlayer gallery of dicalcium phosphate methanol and dicalcium phosphate formamide.
9. The HLCP according to any one of items 3 to 8, wherein in the dicalcium phosphate methanol and in the dicalcium phosphate formamide the distance O^Me to O^HLCP and O^Fm to O^HLCP is inbetween 0.25 and 0.32 nm.
10. The HLCP according to any one of items 1 to 9, wherein the HLCP is non-porous.
11. The HLCP according to any one of items 1 to 10, wherein the at least one organic active substance is selected from the group consisting of antibiotics, antiphlogistics, drugs for the treatment of bone or dental diseases, drugs to promote regeneration of bone or dental tissue, and drugs to prevent rejection of bone or dental implants.
12. The HLCP according to item 11, wherein the at least one organic active substance is selected from the group consisting of hydrogen peroxide, triclosan, penicillin, povidone iodine, amoxicillin, clindamycin, doxycycline, gentamycin, chlorhexidine, rifamycin, vancomycin, LL-27, parathormone, BMP-2, BMP-7, TGF-beta, BMP-3, siRNA, romosozumab, and mixtures thereof.
13. The HLCP according to any one of items 1 to 12 for use in bone tissue engineering or regenerative medicine, as or in bioceramics, as or in biocomposites, as or in bone scaffolds, as or in implants, as or in implant coatings, as or in CaP printable powders or liquids or as additives, and/or as an active substance carrier.
14. The HLCP according to item 13, wherein the implants are bone or dental implants or the implant coating is a bone or dental implant coating.
15. The HLCP according to items 13 or 14 comprising at least one organic active substance selected from the group consisting of antibiotics, antiphlogistics, drugs for the treatment of bone or dental diseases, drugs to promote regeneration of bone or dental tissue, and drugs to prevent rejection of bone or dental implants.
16. The HLCP according to item 15, wherein the at least one organic active substance is selected from the group consisting of hydrogen peroxide, triclosan, penicillin, povidone iodine, amoxicillin, clindamycin, doxycycline, gentamycin, chlorhexidine, rifamycin, vancomycin, LL-27, parathormone, BMP-2, BMP-7, TGF-beta, BMP-3, siRNA, romosozumab, and mixtures thereof.
17. The HLCP according to anyone of items 3 to 7, wherein the at least one organic solvent molecule is dimethyl sulfoxide for use as a medicament with analgesic, antiphlogistic, vasodilatory, and anti-edematous effects.
18. Use of an HLCP according to any one of items 1 to 12 in bone tissue engineering or regenerative medicine as or in bioceramics, as or in biocomposites, as or in bone scaffolds, as or in implants, as or in implant coatings, as or in CaP printable powders or liquids or as additives and/or as an active substance carrier.
19. The use according to item 18, wherein the implants are bone or dental implants or the implant coating is a bone or dental implant coating.
20. The use according to items 18 or 19, wherein the HLCP comprises at least one organic active substance selected from the group consisting of antibiotics, antiphlogistics, drugs for the treatment of bone or dental diseases, drugs to promote regeneration of bone or dental tissue, and drugs to prevent rejection of bone or dental implants.
21. The use according to item 20, wherein the at least one organic active substance is selected from the group consisting of hydrogen peroxide, triclosan, penicillin, povidone iodine, amoxicillin, clindamycin, doxycycline, gentamycin, chlorhexidine, rifamycin, vancomycin, LL-27, parathormone, BMP-2, BMP-7, TGF-beta, BMP-3, siRNA, romosozumab, and mixtures thereof.
22. Use of an HLCP according to any one of items 3 to 7, wherein the HLCP comprises dimethyl sulfoxide as the at least one organic solvent molecule, as a medicament with analgesic, antiphlogistic, vasodilatory, and anti-edematous effects.
23. A method for preventing or reducing bacterial infections, preventing or reducing inflammations, treating bone or dental diseases, promoting the regeneration of bone or dental tissue, and/or preventing rejection of bone or dental implants comprising the step of:
   implanting a material comprising an HLCP according to any one of items 1 to 12 carrying at least one organic active substance selected from the group consisting of antibiotics, antiphlogistics, drugs for the treatment of bone or dental diseases, drugs to promote regeneration of bone or dental tissue, and drugs to prevent rejection of bone or dental implants.
24. The method according to item 23, wherein the at least one organic active substance is selected from the group consisting of hydrogen peroxide, triclosan, penicilin, povidone iodine, amoxicillin, clindamycin, doxycycline, gentamycin, chlorhexidine, rifamycin, vancomycin, LL-27, parathormone, BMP-2, BMP-7, TGF-beta, BMP-3, siRNA, romosozumab, and mixtures thereof.
25. A method for treating pain, inflammations, hypertension, and/or edema comprising the step of:
   implanting a material comprising an HLCP according to any one of items 3 to 7, wherein the HLCP comprises dimethyl sulfoxide as the at least one organic solvent molecule.
26. A method for the manufacture of an HLCP (according to any one of items 1 to 12) comprising the step of:
   incubating a crystalline or precipitate calcium phosphate material with at least one organic solvent or at least one organic active substance or a mixture of both comprising 0 to 15 vol.% of water.
27. The method of item 26, wherein the calcium phosphate material is selected from the group consisting of amorphous dicalcium phosphate containing 0-2 moles of water per mole of dicalcium phosphate, crystalline dicalcium phosphate monohydrate, and dicalcium phosphate dihydrate.
28. The method of items 26 or 27, wherein the at least one organic solvent is a polar or dipole organic solvent.
29. The method according to any one of items 26 to 28, wherein the at least one organic solvent is selected from the group consisting of methanol, formamide, dimethyl sulfoxide, dimethylformamide, glycerol, ethylene glycol, and ethanol.
30. The method according to any one of items 26 to 29, wherein for the manufacture of dicalcium phosphate methanol (CaHPO₄ • CH₃OH) a calcium phosphate material selected from the group consisting of amorphous dicalcium phosphate containing 0-2 moles of water per mole of dicalcium phosphate, crystalline dicalcium phosphate monohydrate, and dicalcium phosphate dihydrate is incubated with methanol as an organic solvent, wherein the methanol comprises 0 to 15 vol.% water.
31. The method according to items 26 to 29, wherein for the manufacture of dicalcium phosphate formamide (CaHPO₄ • HCONH₂) a calcium phosphate material selected from the group consisting of amorphous dicalcium phosphate containing 0-2 moles of water per mole of dicalcium phosphate, crystalline dicalcium phosphate monohydrate, and dicalcium phosphate dihydrate is incubated with formamide as an organic solvent, wherein the formamide comprises 0 to 15 vol.% water.
32. The method according to items 26 to 29, wherein for the manufacture of dicalcium phosphate dimethyl sulfoxide (CaHPO₄ • (CH₃)₂SO) a calcium phosphate material selected from the group consisting of amorphous dicalcium phosphate containing 0-2 moles of water per mole of dicalcium phosphate, crystalline dicalcium phosphate monohydrate, and dicalcium phosphate dihydrate is incubated with dimethyl sulfoxide as an organic solvent, wherein the dimethyl sulfoxide comprises 0 to 15 vol.% water.
33. The method according to items 26 to 29, wherein for the manufacture of dicalcium phosphate dimethylformamide (CaHPO₄ • HCON(CH₃)₂) a calcium phosphate material selected from the group consisting of amorphous dicalcium phosphate containing 0-2 moles of water per mole of dicalcium phosphate, crystalline dicalcium phosphate monohydrate, and dicalcium phosphate dihydrate is incubated with dimethylformamide as an organic solvent, wherein the dimethylformamide comprises 0 to 15 vol.% water.
34. The method according to items 26 to 29, wherein for the manufacture of dicalcium phosphate glycerol (CaHPO₄ • HO-CH₂-CH(OH)-CH₂-OH) a calcium phosphate material selected from the group consisting of amorphous dicalcium phosphate containing 0-2 moles of water per mole of dicalcium phosphate, crystalline dicalcium phosphate monohydrate, and dicalcium phosphate dihydrate is incubated with glycerol as an organic solvent, wherein the glycerol comprises 0 to 15 vol.% water.
35. The method according to items 26 to 29, wherein for the manufacture of dicalcium phosphate ethylene glycol (CaHPO₄ • HO-CH₂-CH₂-OH) a calcium phosphate material selected from the group consisting of amorphous dicalcium phosphate containing 0-2 moles of water per mole of dicalcium phosphate, crystalline dicalcium phosphate monohydrate, and dicalcium phosphate dihydrate is incubated with ethylene glycol as an organic solvent, wherein the ethylene glycol comprises 0 to 15 vol.% water.
36. The method according to items 26 to 29, wherein for the manufacture of dicalcium phosphate ethanol (CaHPO₄ • C₂H₅OH) a calcium phosphate material selected from the group consisting of amorphous dicalcium phosphate containing 0-2 moles of water per mole of dicalcium phosphate, crystalline dicalcium phosphate monohydrate, and dicalcium phosphate dihydrate is incubated with ethanol as an organic solvent, wherein the ethanol comprises 0 to 15 vol.% water.
37. The method according to any one of items 26 to 36, wherein the incubation is carried out at 25 ± 5 °C for about 24 hours, preferably with shaking at about 90 rpm.
38. A method for the conversion of one HLCP into another HLCP, wherein an HLCP according to any one of items 1 to 12 is incubated with a different organic solvent.
39. The method according to item 38, wherein the incubation is carried out at 25 ± 5 °C for about 24 hours, preferably with shaking at about 90 rpm.

Various modifications and variations of the invention will be apparent to those skilled in the art without departing from the scope of invention. Although the invention has been described in connection with specific preferred embodiments, it should be understood that the invention as claimed should not be unduly limited to such specific embodiments. Indeed, various modifications of the described modes for carrying out the invention which are obvious to those skilled in the art in the relevant fields are intended to be covered by the present invention.

### BRIEF DESCRIPTION OF THE FIGURES

The following Figures are merely illustrative of the present invention and should not be construed to limit the scope of the invention as indicated by the appended claims in any way.
**FIGURE 1****:** Shows XRD patterns of the captured phases by quenching at different time with methanol (MeOH) or 1:1 v/v methanol:ethanol (MeOH:EtOH).
**FIGURE 2****:** Shows XRD patterns of the products when the captured ACHP was incubated for 24 hours in different methanol (MeOH) solutions.
**FIGURE 3****:** Shows HLCP-Me prepared by incubating ACHP in a methanol solution (the ACHP diffractogram is shown at the bottom, plots are stacked according to increasing incubation time towards the topmost diffractogram).
**FIGURE 4****:** Shows HLCP-Me prepared by incubating DPCM in a methanol solution (the DCPM diffractogram is shown at the bottom, plots are stacked according to increasing incubation time towards the topmost diffractogram).
**FIGURE 5****:** Shows SEM images of HLCP-Me, HLCP-Fm, HLCP-DMSO, and HLCP-DMF. When viewed in landscape format: Top left HLCP-Me, top right HLCP-Fm, bottom left HLCP-DMSO, bottom right HLCP-DMF
**FIGURE 6****:** Shows XRD spectra of HLCP-Me, HLCP-Fm, HLCP-DMSO, and HLCP-DMF.
**FIGURE 7****:** Shows electron diffraction from a crystal of HLCP-Me and HLCP-Fm. Top row HLCP-Me, bottom row HLCP-Fm.
**FIGURE 8****:** Shows FTIR spectra of HLCP-Me, HLCP-Fm, HLCP-DMSO and HLCP-DMF.
**FIGURE 9****:** Shows crystalline structures of HLCP-Me (a, c and e) and HLCP-Fm (b, d and f) viewed along *a*-direction (a and b), *b*-direction (c and d), and *c*-direction (e and f). Ca atoms: large grey, P: large black, O: medium grey, C: medium black and N: small grey.
**FIGURE 10****:** Shows the optimized coordinate of HLCP-Me and HLCP-Fm with hydrogen bonds (dashed lines).
**FIGURE 11****:** Shows the phase transformation between HLCP-Me, HLCP-Fm, HLCP-DMSO, and HLCP-DMF.
**FIGURES 12 to 15****:** Show Pawley fits of the PXRD data from HLCP-Me (Figure 12), HLCP-Fm (Figure 13), HLCP-DMSO (Figure 14), and HLCP-DMF (Figure 15). Each diffraction pattern is split into two angular ranges (top and bottom). The diffraction patterns show observed data (top, black), fit middle (dark grey) and residuals (bottom, light grey).
**FIGURES 16** **and** **17****:** Show Rietveld refinements of HLCP-Me and DCPM-Fm. Each diffraction pattern is split into two angular ranges (top and bottom). The diffraction patterns show observed data (top, black), fit middle (dark grey) and residuals (bottom, light grey).

### EXAMPLES

The practical aspects and examples described below are for illustrative purposes only and are not intended to limit the invention described above in any way.

### The preparation of HLCPs

Information about all chemicals used in the present invention is summarized in Table 1. HLCPs were prepared by incubating amorphous calcium hydrogen phosphate (ACHP) in different organic solutions.

For capturing ACHP from the reaction between the calcium and biphosphate ions, 0.142 g Na₂HPO₄ (1.00 mmol) and 0.185g CaCl₂ (1.67 mmol) were respectively dissolved in 3 mL and 2 mL deionized water, the two solutions were quickly mixed in a 50 mL beaker under vigorous stirring (ca. 400 rpm), and after 3 seconds, a 34 mL mixture of 1:1 (v/v) methanol:ethanol was added to quench the reaction (no later than 15 seconds otherwise other phases instead of ACHP would be captured).

The quenched solution was evenly distributed into two centrifuge tubes where a 23 mL mixture of 1:1 v/v methanol:ethanol was reserved in advance, then centrifugation was run immediately until the rotation speed reached 2500 rpm (with no holding time here), and the supernatants were quickly removed.

In order to completely remove water, ethanol was used for another four times of centrifugation, and centrifuge tubes were shaken vigorously to disperse sediments in ethanol before centrifugation started.

The centrifugation was stopped once the speed reached 4500/6000/6000 rpm and 6000 rpm with a holding time of 1 minute for the last time, and after the supernatants were removed, the final sediments of ACHP were introduced in different organic solutions to incubate ACHP in a water-poor environment.

The organic solutions were particularly 0.9 mL water in 35 mL methanol, 2 mL water in 35 mL formamide, 1.5 mL water in 35 mL dimethyl sulfoxide, and 1.5 mL water in 35 mL dimethylformamide, and the incubations were all conducted for several days in an oscillation incubator with a constant shaking speed of 90 rpm at room temperature (25 ± 5 °C).

The final products were separated by centrifuging at 6000 rpm for 1 minute and then dried using a hair-dryer for immediate use or dried in a vacuum oven under 35 °C to avoid moisture.

It should be noted that the whole process of quenching and centrifuging usually could be done within 3 minutes when all solutions were ready for use. Such quick operations could make sure that the water in the initial reacting system was removed completely and as soon as possible, otherwise other phases would form from the aqueous reaction between calcium and biphosphate ions.

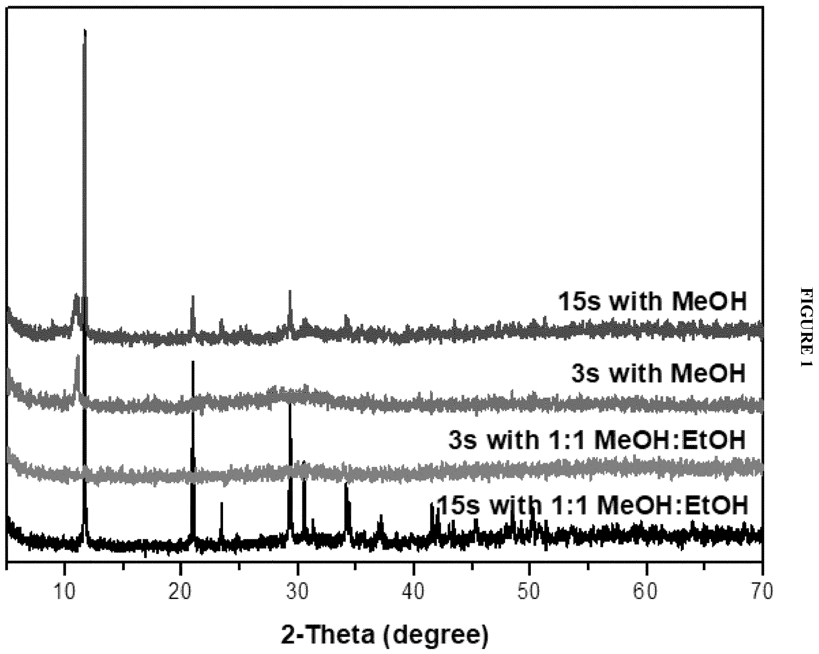

Based on the results of the inventors, when the aqueous reaction was quenched with the mixture of methanol and ethanol at different time points, the captured phase was, for example, only ACHP at the 3rd second but contained dicalcium phosphate dihydrate (DCPD) at the 15th second, and when quenched with only methanol, the captured phase still was ACHP at the 3rd second and DCPD at the 15th second, but also contained dicalcium phosphate monohydrate (DCPM) at both the 3rd and 15th second (Figure 1).

This indicated that the mixture of methanol and ethanol was more effective in stopping the aqueous reaction between calcium and biphosphate ions, and thus, the quenching procedure was crucial for capturing the pure ACHP phase. Besides, DCPM and DCPD impurities could form at the 3rd second if the mixture of methanol and ethanol was far less used at the quenching step or water was not rapidly and completely excluded during the centrifuging process. Therefore, it is recommended that at least 34 mL of the mixture should be added in the initial 5 mL water system to effectively quench the aqueous reaction, and the quenching and centrifuging operations should be done within 3 minutes (as quickly as possible).

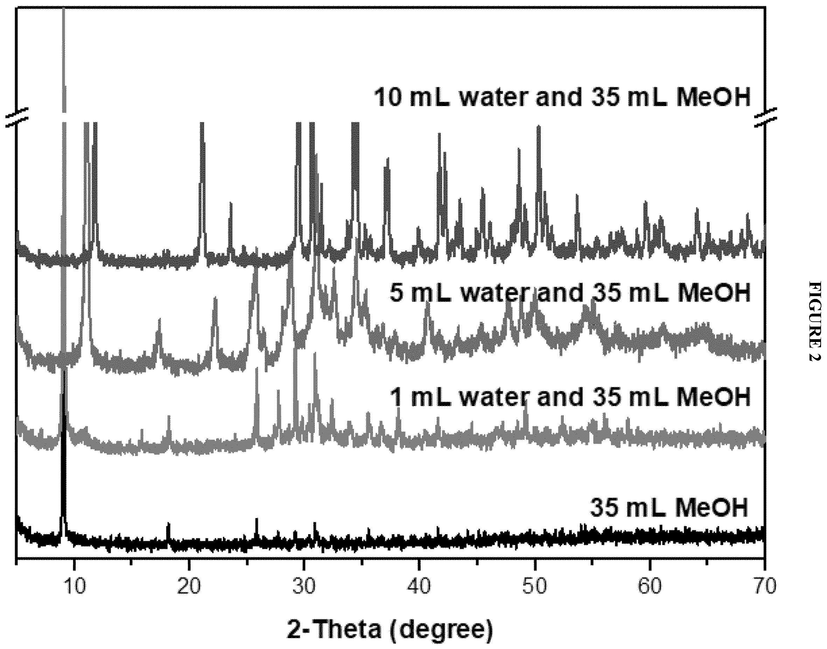

Regarding the incubation of ACHP in organic solutions, the final products could be influenced by the content of water. Taking the incubations of ACHP in methanol solutions (24 hours) as examples, the addition of 10, 5 and 1 mL water in 35 mL methanol, respectively, resulted in DCPD, DCPM, and HLCP-Me; and in pure methanol, HLCP-Me was also produced but exhibited a PXRD diffraction pattern indicative of lower crystallinity than in the presence of 1 mL water (Figure 2).

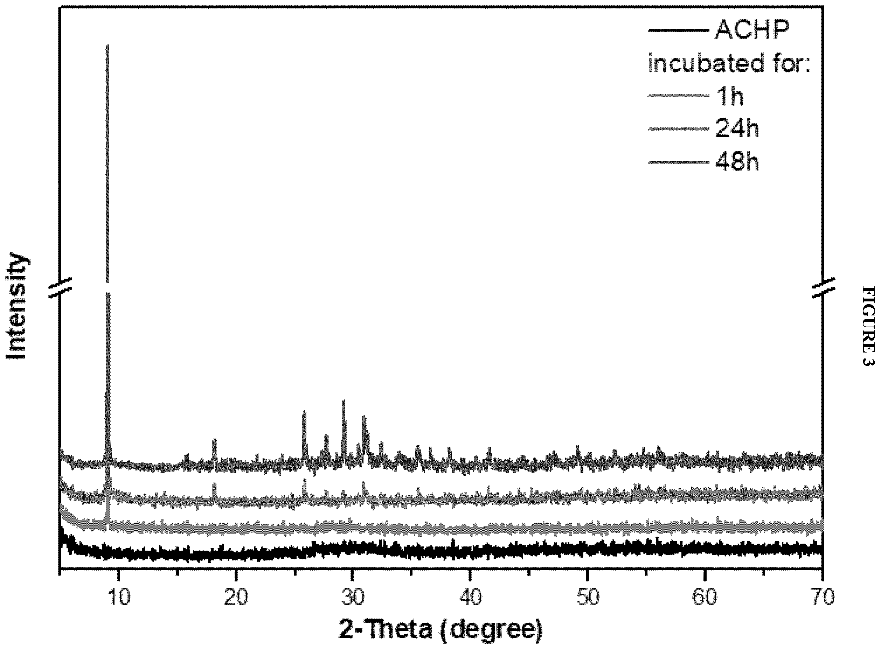

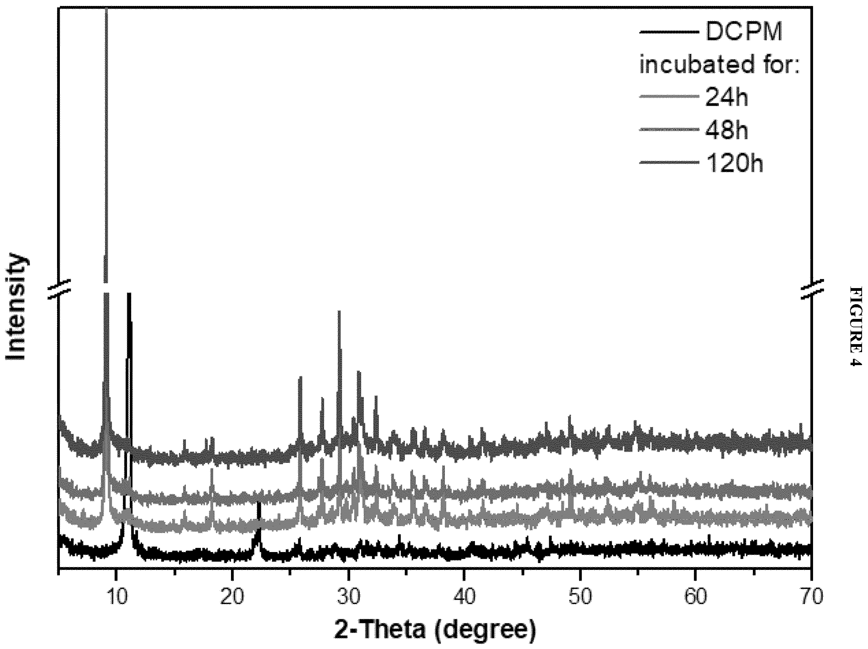

In fact, HLCP-Me was even detected by XRD just after 1 hour of incubation in the presence of 1 mL water in 35 mL methanol (Figure 3). This indicated that a small amount of water (ca. < 3% v/v) could facilitate the crystallization of HLCP-Me, while DCPM would form if the water content was about 15% (v/v) in methanol²² and DCPD would form in a water-rich environment (>> 15%). It is worth pointing out that by well-controlling a water-poor environment, HLCP-Me was also produced by incubating DCPM in a mixture of 35 mL methanol and 0.9 mL water for 24 hours or longer (Figure 4).

For preparing HLCP-Me, HLCP-Fm, HLCP-DMSO and HLCP-DMF, it is recommended to introduce 0.9, 2, 1.5, and 1.5 mL of water in the 35 mL of methanol, formamide, dimethyl sulfoxide, and dimethylformamide, respectively.

**Table 1: Chemical information.**

| Chemicals | Formula | CAS No. | Quality | Supplier |
|---|---|---|---|---|
| Sodium phosphate dibasic | Na₂HPO₄ | 7558-79-4 | 99.0% | Sigma-Aldrich |
| Calcium chloride anhydrous | CaCl₂ | 10043-52-4 | 93.0% | Sigma-Aldrich |
| Ethanol | CH₃CH₂OH | 64-17-5 | 99.5% | Kiiltoclean A/S, Sweden |
| Methanol | CH₃OH | 67-56-1 | 99.8% | Honeywell Riedel de Haën^{™} |
| Formamide | CH₃NO | 75-12-7 | 98.5% | Tokyo Chemical Industry Co., LTD |
| Dimethyl sulfoxide | (CH₃)₂SO | 67-68-5 | 99.9% | VWR Chemicals |
| N,N-Dimethylformamide | C₃H₇NO | 68-12-2 | 99.8% | Honeywell Riedel de Haën^{™} |

### Structural characterization of HLCPs

The HLCPs dried in a vacuum oven under 35 °C for one day were characterized by using a scanning electron microscope, infrared spectroscopy, and X-ray powder diffraction. The scanning electron microscope JEOL JSM-7000F was equipped with a Schottky field emission gun and offers high resolution and large probe currents at small probe diameters for microstructure determination of HLCPs.

The IR spectra of HLCPs were recorded on a Varian 670-IR FTIR spectrometer using a Specac Goldengate single reflection attenuated total reflection (ATR) accessory with a diamond ATR element for quick measurements and no sample preparation is needed. The ATR spectra were recorded in the wavelength range of 4000-400 cm⁻¹ with the background spectrum of air, and the reflectance data were converted into transmittance for presentation and analysis.

The X-ray powder diffraction patterns were recorded using a Panalytical X'Pert alpha 1 with a focusing Johansson Ge monochromator producing pure Cu-Kα1 radiation at (λ = 1.540598 Å). For general characterization, diffraction patterns were measured in the 2θ range of 5-70 ° within 10 minutes, and for Rietveld refinements, high-quality data were recorded more than 10 hours on fine powders of the sample filled in a 0.5 mm silica capillary.

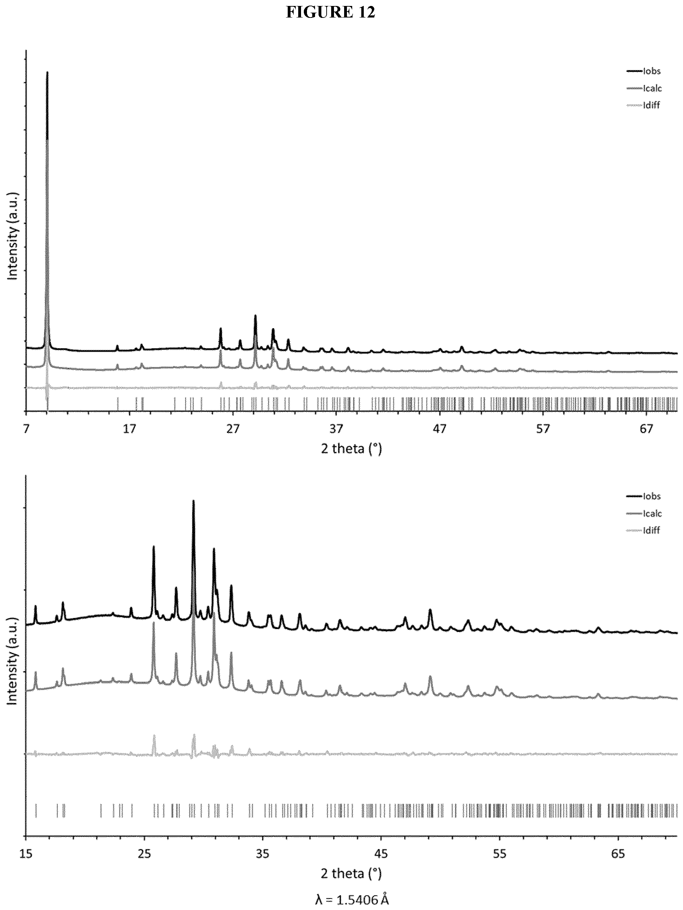

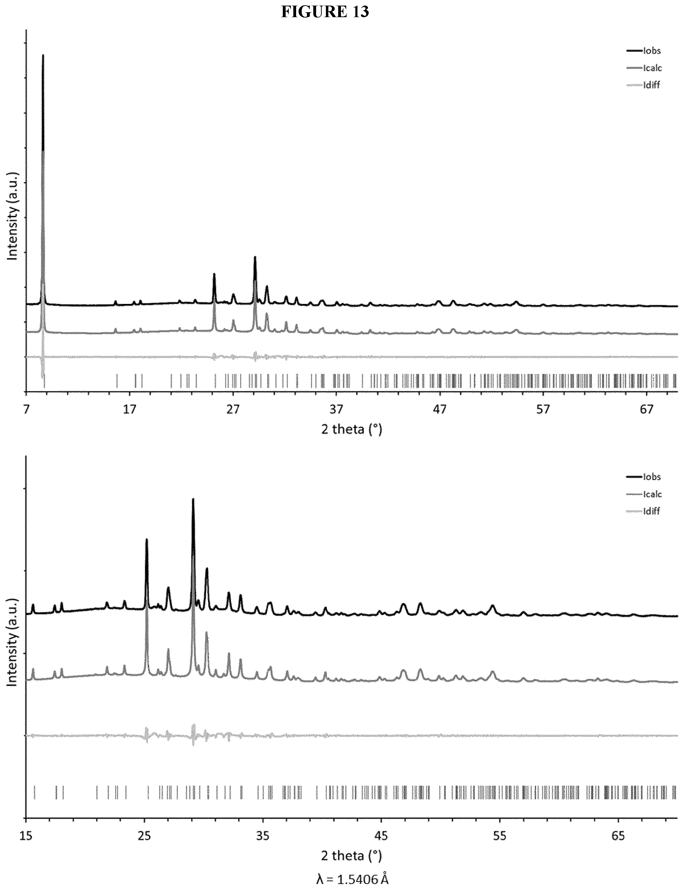

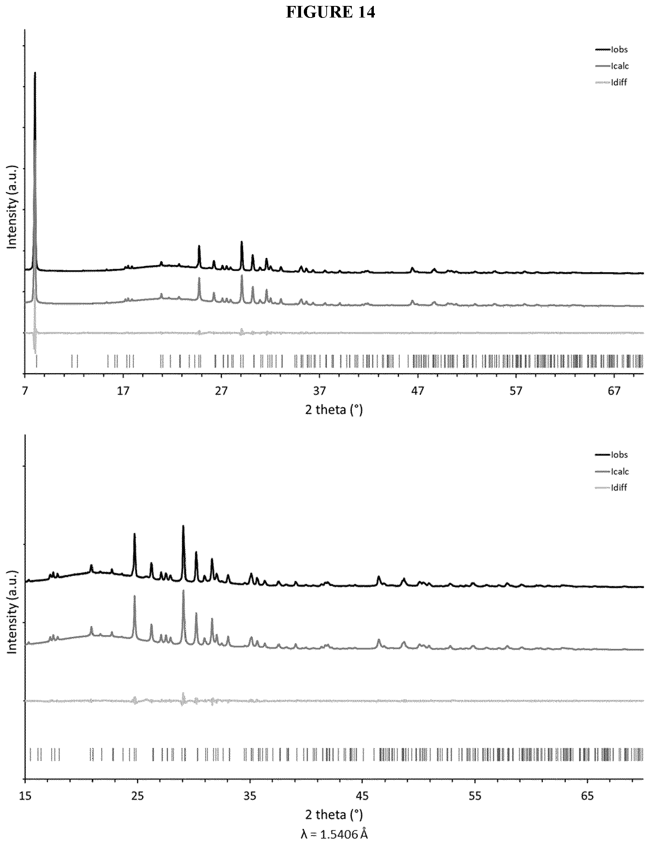

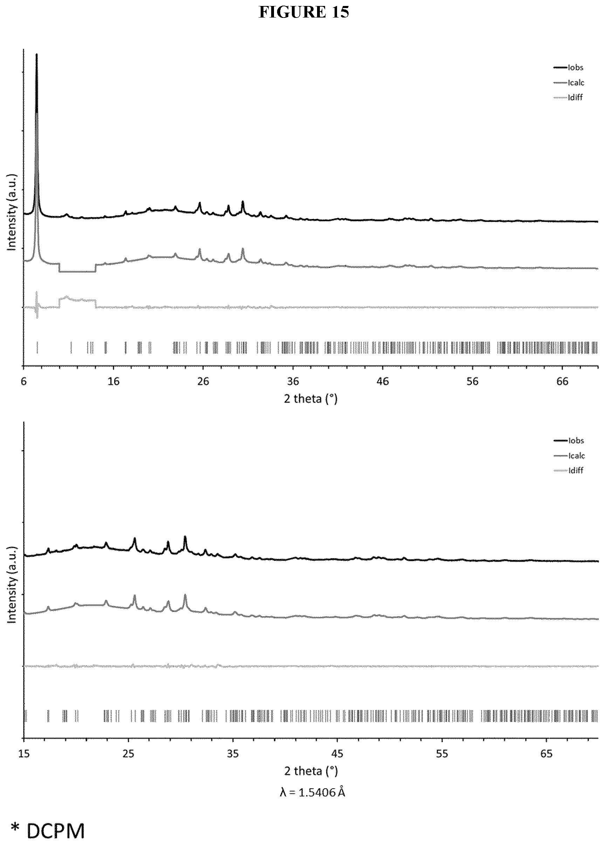

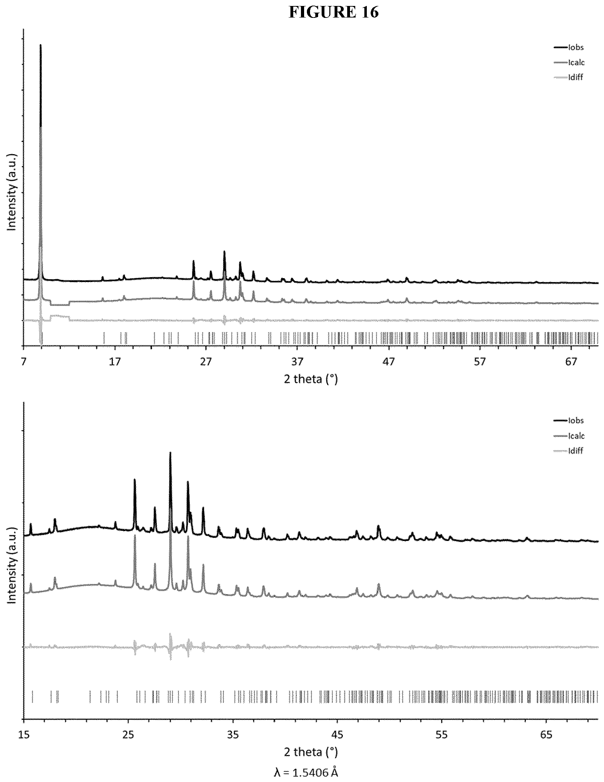

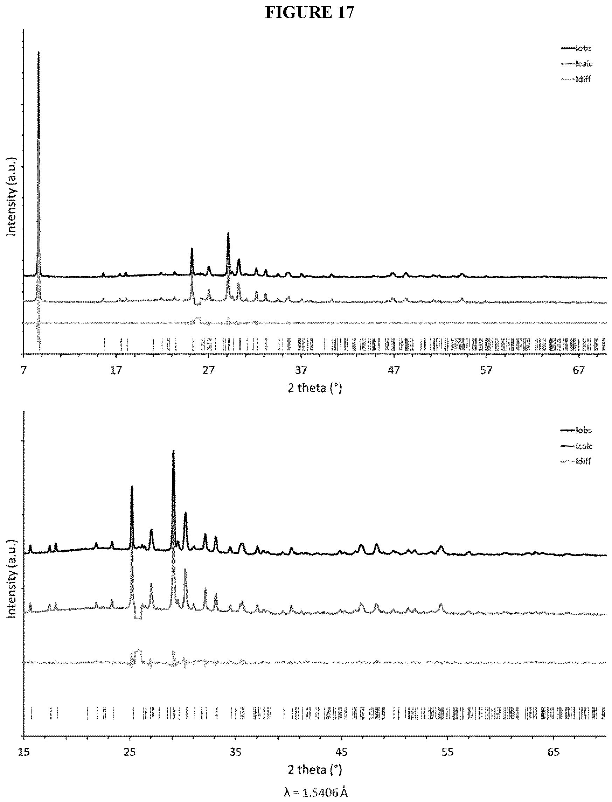

The Rietveld refinement was performed using the software Topas version 5 (Coelho, A. TOPAS-Academic V5. Coelho Software. 2012). The refinement was performed using isotropic atomic displacement parameters, a pseudo-Voigt peak shape function, and seven parameters to fit the background. No restraints on the geometry of the structure were used. Figures 12 to 15 show Pawley fits of the PXRD data from HLCP-Me, HLCP-Fm, HLCP-DMSO, and HLCP-DMF (the material contains sone impurity peaks), and Figures 16 and 17 show Rietveld refinements of HLCP-Me. The crystallographic data for HLCP-Me are shown in Table 2.

**Table 2: crystallographic data for HLCP-Me.**

| | HLCP-Me |
|---|---|
| Crystal system | Monoclinic |
| Space group (No.) | *P*2₁/*c* (14) |
| a, nm | 1.0153 |
| b, nm | 0.6814 |
| c, nm | 0.7725 |
| α,° | 90 |
| β,° | 106.001 |
| γ,° | 90 |
| R_{wp} | 0.0816 |

### Continuous rotation electron diffraction

After the incubation of ACHP in different organic solutions for a certain period, half of the incubating solutions were centrifuged to collect HLCPs for XRD confirmation, and a droplet of the other half solutions was transferred onto a copper grid covered by a holey carbon film. After gently drying by a hair-dryer, the grid was mounted to a single-tilt cryo-transfer tomography holder, Gatan Type 914, and cooled by liquid nitrogen to ~ -176 °C for further operations.

A JEOL JEM-2100 transmission electron microscope (TEM) was operated at an accelerating voltage of 200 kV for recording continuous rotation electron diffraction (cRED). The cRED data were acquired by continuously tilting the goniometer with a tilt speed of 1.12°/s with a high-speed hybrid detection camera Timepix Quad (ASI), and the crystal on focus was meanwhile tracked by sequential defocusing of the intermediate lens using the software Instamatic¹⁸.

The datasets were processed using X-ray Detector Software (XDS) in order to extract intensities for structure solution and refinement. The reconstructed reciprocal lattice of HLCPs could be indexed by monoclinic unit cells and systematic extinctions indicated that the space group was *P*2₁/c. The structures were solved using the software SHELXT¹⁹ and the subsequent least-squares refinements were performed in SHELXL-97²⁰ using atomic scattering factors for electrons extracted from SIR2014²¹.

The structure solutions directly identified 7 peaks identified as 1 Ca, 1 P, and 5 O. The least squares refinement converged without the use of any restraints and with reasonable isotropic atomic displacement parameters to an R1 residual of 0.260, using 29 refinement parameters. No restraints on the geometry of the structure were used during the refinement. Details regarding the cRED data collection, as well as the refinement, can be found in Table 4. Details of the structure after refinement against cRED data are available from the Cambridge Crystallographic Data Center with deposition code CCDC 1961466. cRED data were also collected from the sample of DCPM prepared in humid air. Structure solution resulted in the same structure as for the sample prepared by mixed solvent and the refinement converged with an R1 residual of 0.256. Table 3 shows the statistics of the least-squares refinement of HCPL-Me against cRED data.

**Table 3: Statistics of the least-squares refinement of HCPL-Me against cRED data.**

| Crystal system | Monoclinic |
|---|---|
| Space group (No.) | *P*2₁/*c* (14) |
| a, nm | 1.0153 |
| b, nm | 0.6814 |
| c, nm | 0.7725 |
| α, ° | 90 |
| β, ° | 106.001 |
| γ, ° | 90 |
| Volume, Å³ | 521.66 |
| λ, Å | 0.0251 |
| Tilt range (°) | -50.9 to +41.50 |
| No. cRED frames | 350 |
| Tilt step per frame (° ) | 0.264 |
| Exposure time per frame (s) | 0.5 |
| Completeness, % | 50.6 |
| No. refined parameters | 33 |
| Resolution, Å | 0.87 |
| No. restraints | 0 |
| Rᵢₙₜ | 0.06 |
| No. symmetry independent reflections | 454 |
| R₁ | 0.241 |

### Phase transformation between HLCPs

The phase transformation between HLCPs was studied with an initial phase prepared from ACHP. Specifically, the initial phase of HLCP-Me was prepared by incubating ACHP in a methanol solution for 1 day (24 hours), and HLCP-Me which was confirmed by XRD was then transferred in the dimethylformamide solution and incubated for 1 day to study the transformation from HLCP-Me to HLCP-DMF; similarly, the initial HLCP-DMF prepared from ACHP was transferred in the dimethyl sulfoxide solution and incubated for 1 day to study the transformation from HLCP-DMF to HLCP-DMSO, and likewise, the initial HLCP-DMSO prepared from ACHP was incubated in the formamide solution for 1 day to study the transformation from HLCP-DMSO to HLCP-Fm. Here the methanol, dimethylformamide, dimethyl sulfoxide, and formamide solution were, respectively, made up by the pure organic solvent (35 mL) with 0.9, 1.5, 1.5 and 2 mL deionized water as recommended above. Due to the short period of incubation, the phase transformation could not be complete but the co-existence of the initial phase and the targeting phase can be expected.

### Model drug loading experiments

Sulfanilamide (SA) antibiotic was chosen as a model drug to study the drug loading efficacy of HLCPs. The solubility of SA is 11.4 wt. % (ca. 90.5 mg/mL) in methanol at 25 °C, 2.8 wt. % (ca. 30.8 mg/mL) in DMSO and 4.4 wt. % (ca. 8.8 mg/mL) in DMF (21.4 wt. % (ca. 215 mg/mL) in EtOH, 7.525g in 35 mL EtOH) at 20 °C, but it would slightly decrease with the presence and increase of water. Therefore, the saturated solutions of SA were prepared by dissolving 3.168, 1.085, and 0.308 g SA, respectively, in the methanol, DMSO, and DMSO solutions, which contain a small amount of deionized water (0.9, 1.5, and 1.5 mL), and the final solutions were a little over-saturated. The SA solutions were then used to incubate ACHP for several days by shaking and standing at room temperature.

### Results

The HLCP biomaterials possess the same layers of calcium hydrogen phosphate as dicalcium phosphate monohydrate (DCPM)¹⁷, while incorporating organics in the interlayer galleries instead of water molecules in the DCPM.

Examples of organic solvents that can be used for the preparation of the inventive HLCPs are methanol (HLCP-Me), formamide (HLCP-Fm), dimethylformamide (HLCP-DMF), and dimethyl sulfoxide (HLCP-DMSO).

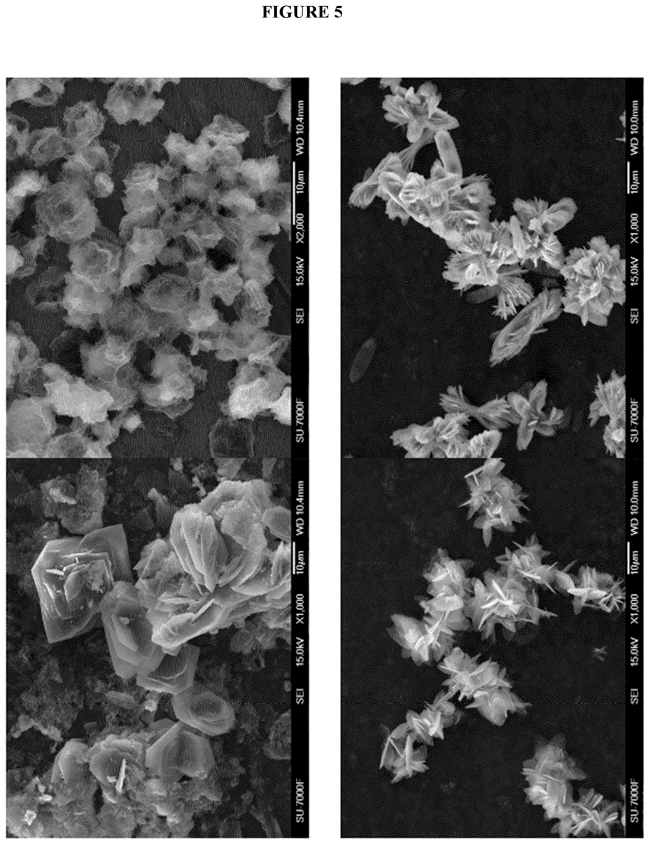

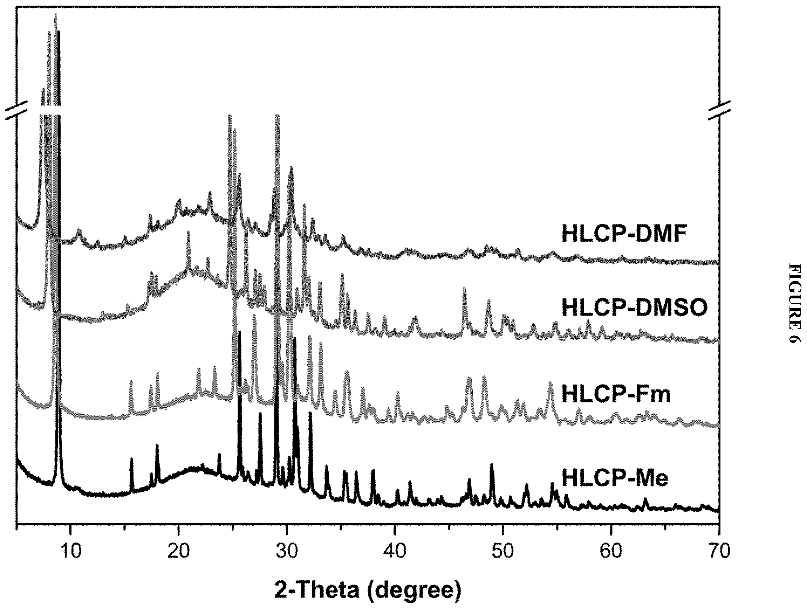

As observed by scanning electron microscope (SEM), the above HLCPs exhibited flake-shaped morphologies with the difference in size and thickness, and more concretely, HLCP-Me microcrystals are much larger and thicker than HLCP-Fm, HLCP-DMF, and HLCP-DMSO (Figure 5). The distinct crystalline characters were confirmed by X-ray powder diffraction (XRPD). The highest intensity reflection, describing the interlayer distances of the HLCP phases, can be derived from the XRPD patterns (Figure 6) to correspond to a d-spacing of 0.98, 0.101, 0.108, and 0.115 nm for HLCP-Me, HLCP-Fm, HLCP-DMSO, and HLCP-DMF, respectively.

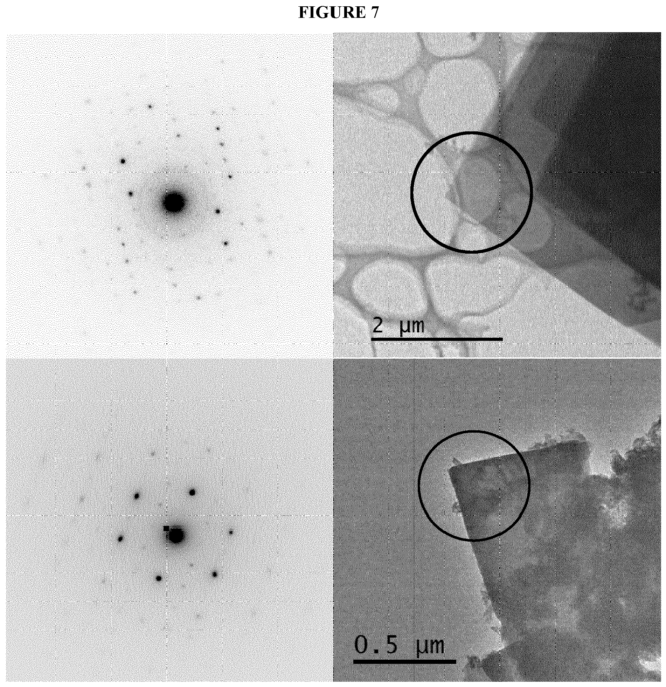

The continuous Rotation Electron Diffraction technique (cRED) was further employed for structure determination, by which a series of selected-area electron-diffraction patterns from micrometer-sized crystals (Figure 7) were collected using a transmission electron microscope (TEM).

The reconstructed three-dimensional reciprocal lattice of HLCP-Me could be indexed using a monoclinic unit cell and space group P2₁/c. Based on the cRED data the structure could be determined ab-initio and refined using least-squares refinement, see Table 3.

The structure was further refined against XRPD data, see Table 4. The structure contains one unique Ca²⁺ and one unique phosphate ion forming a similar layer as in DCPM. Between the layers, a methanol molecule can be found coordinating with the Ca²⁺ ion. cRED data acquired from the HLCP-Fm, HLCP-DMF, and HLCP-DMSO all showed reciprocal lattices consistent with a similar layer.

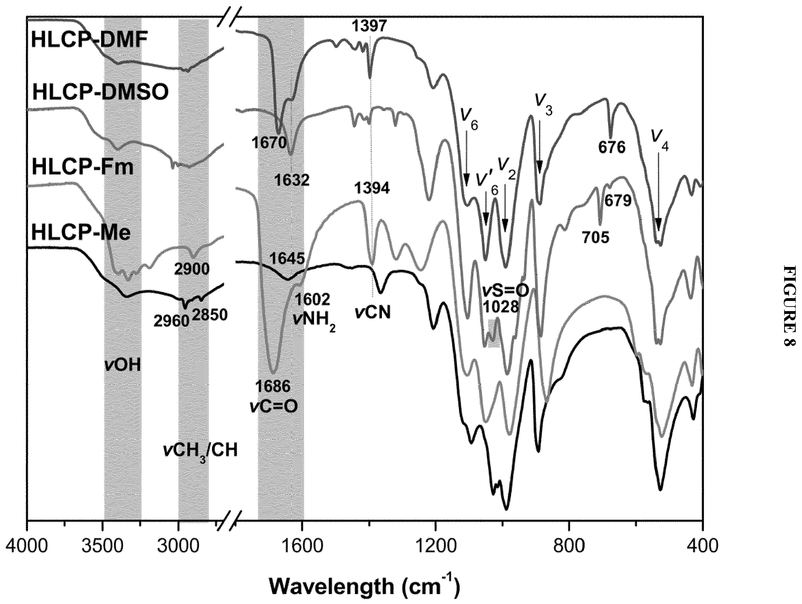

Pawley fits against the XRPD data of each phase confirmed the monoclinic structures with very similar unit cell dimensions along b and c and different interlayer distances (Table 4 and Figure 8). This is consistent with the intercalation of each of the four molecules (Me, Fm, DMSO, and DMF) between identical calcium phosphate layers. Based on the structural similarities, symmetry, and unit cell volume it is most likely that one organic molecule is intercalated per calcium and phosphate ion, and consequently the chemical formulae of Ca-HPO₄•CH₃OH, CaHPO₄•CH₃NO, CaHPO₄•(CH₃)₂SO, and CaHPO₄•CH₃NCHO were proposed for HLCP-Me, HLCP-Fm, HLCP-DMF, and HLCP-DMSO, respectively.

**Table 4: Unit cell parameters of HLCP phases as determined from PXRD in comparison with the published DCPM.**

| | DCPM | HLCP-Me | HLCP-Fm | HLCP-DMSO | HLCP-DMF |
|---|---|---|---|---|---|
| *a (Å)* | 8.00 | 10.153(3) | 10.470(3) | 11.242(3) | 12.09(3) |
| *b (Å)* | 6.79 | 6.814(2) | 6.784(2) | 6.759(1) | 6.77(2) |
| *c (Å)* | 7.78 | 7.725(3) | 7.879(2) | 7.803(2) | 7.82(2) |
| *α (°)* | 90 | 90 | 90 | 90 | 90 |
| *β (°)* | 91.53 | 106.001(4) | 105.578(4) | 106.065(3) | 107.24(2) |
| *γ (°)* | 90 | 90 | 90 | 90 | 90 |
| *SG* | *P*2₁/*c* | *P*2₁/*c* | *P*2₁/*c* | *P*2₁/*c* | *P*2₁/*c* |

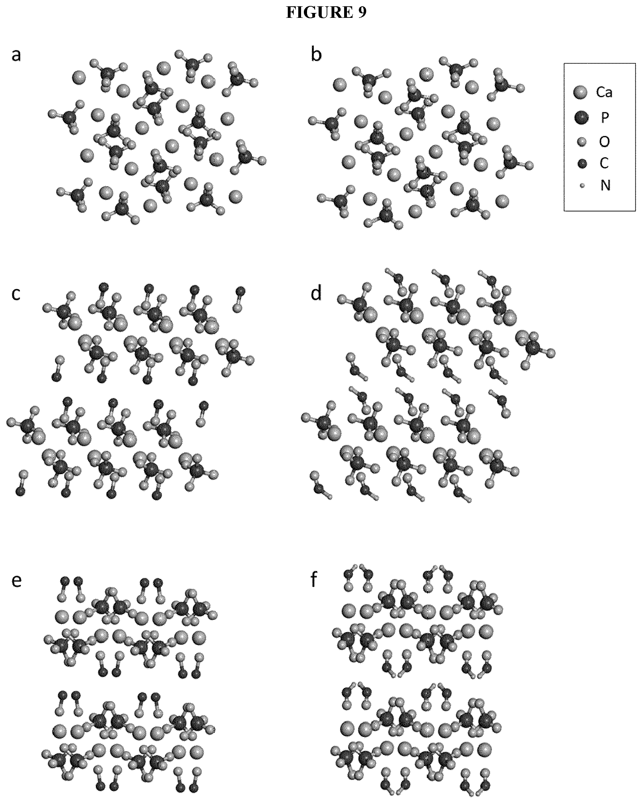

In order to gain more confidence, the structural incorporation of methanol, formamide, dimethylformamide, and dimethyl sulfoxide was further studied by Fourier-transform infrared spectroscopy (FTIR). As shown in Figure 9, the vibration bands of HPO₄²⁻ in HCLP-Me, HLCP-Fm, HLCP-DMSO, and HLCP-DMF are consistent with the v2, v3, v4, v6, and v6' modes, which were identified for the DCPM¹⁰, while the v6 and v6' peaks of HLCP-Me were overlapped with the C-O stretching vibrations of the incorporated methanol (ca. 1053, 1033, 1019 cm⁻¹)¹¹.

In the spectrum of HLCP-Me, two bands at 2960 and 2850 cm⁻¹ respectively corresponded to the asymmetric and symmetric stretching vibrations of the methyl (CH₃) group in methanol¹¹, and one peak at ca. 2900 cm⁻¹ in the spectra of the other three HLCP was attributed to the CH stretching mode of formamide, dimethylformamide, and dimethyl sulfoxide. In the spectrum of HLCP-Fm, the bands at 1686 cm⁻¹ and 1602 cm⁻¹ were respectively attributed to the carbonyl (C=O) stretching and amine (NH₂) bending mode of formamide¹².

In the spectrum of HLCP-DMF, the C=O stretching vibration of dimethylformamide shifted to 1670 cm⁻¹ with the O=C-N bending vibration found at 676 cm^{-1 13,14}. In the spectrum of HLCP-DMSO, the bands at 705 and 679 cm⁻¹ were respectively attributed to the asymmetric and symmetric stretching vibrations of CS group, and the peak at 1028 cm⁻¹ was in the fingerprint region (1000-1059 cm⁻¹) of sulfinyl (S=O) group of dimethyl sulfoxide¹⁵.

The wavenumber of the vibrational mode of the S=O group in dimethyl sulfoxide is highly sensitive to its hydrogen-bonding environment, for instance, the band corresponding to the double hydrogen bond, single hydrogen bond, aggregate, and "free" S=O respectively appears at 1011.8 (1016.3), 1022.5 (1030.2), 1043.9 (1038.3), and 1058.2 (1053.9) cm⁻¹ in the experimental (simulated) spectra¹⁶. Accordingly, the peak at 1028 cm⁻¹ corresponds to a single hydrogen bond environment of S=O group in HLCP-DMSO. It was indicated that the hydrogen bond probably also occurs in HCLP-Me, HLCP-Fm, and HLCP-DMF HLCP-Me, and in such manner, the organics interacted with the CaHPO₄ layer to form HLCP biomaterials.

Given the difficulty in determining hydrogen positions using electron and x-ray diffraction, first principal quantum mechanical calculations were performed to locate hydrogen atoms and to study the hydrogen-bonding interactions between the organics and CaHPO₄ layers.

The structures of HLCP-Me and HLCP-Fm based on cRED and XRPD data were used as initial models of HLCPs, where the PO₄ and organics units were saturated with fair hydrogen atoms at different positions. Geometry optimizations were run with fully relaxed unit cells, and the stability of optimized geometries associated with the choice of hydrogen positions was confirmed with ab initio molecular dynamics.

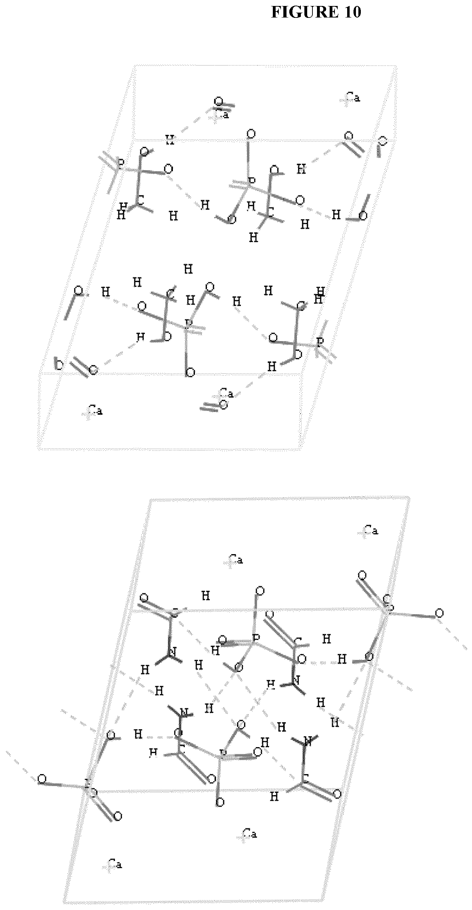

The optimized HLCP-Me and HLCP-Fm are shown in Figure 10, and as expected, the structural order of CaHPO₄ layers in optimized geometries of HLCPs was consistent with that in DCPM and the interlayer distances significantly expanded with respect to the bigger size of organics in comparison to the size of water molecules. The oxygen atom of the methanol and formamide molecules interacts with the calcium ion of the HLCP layer and protons of the organics were oriented toward the oxygen atoms of the HPO₄ anions of the HLCP layers and interacted via hydrogen bonding. The molecular exchange of 4 water molecules in the DCPM unit cell with 4 methanol was proven thermodynamically favorable, as the change in energy (without thermal/vibration correction) was calculated to be about -12.4 kcal/mol in the liquid phase. In comparison, the molecular exchange of water in the unit cell of DCPD with methanol was unfavorable, therefore, the stability could follow the order of DCPD > HLCP-Me (or HLCPs) > DCPM.

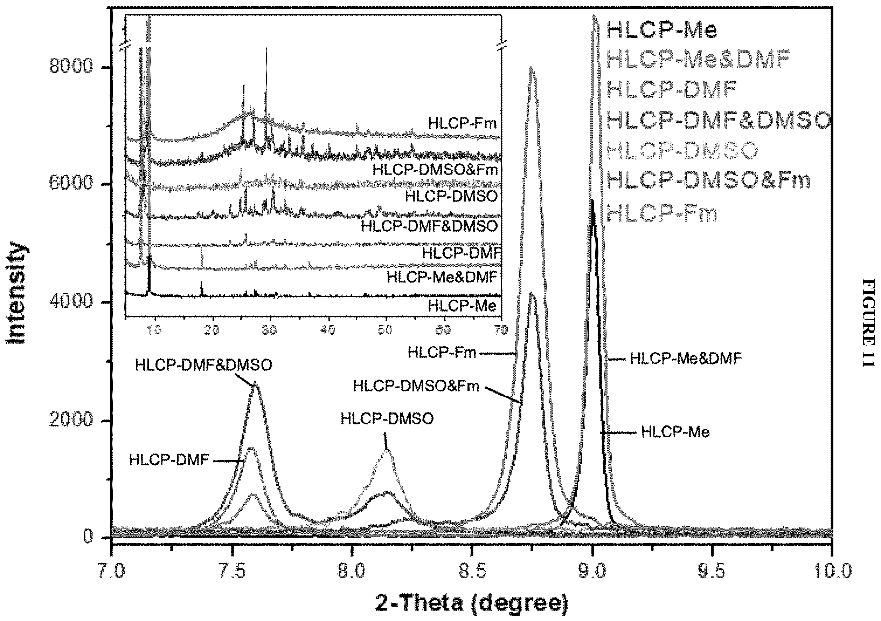

Regarding the HLCPs, phase transformations from HLCP-Me to HLCP-DMF, then to HLCP-DMSO, and finally to HLCP-Fm were demonstrated in Fig. 11, when the incubating solvents were changed from methanol to dimethylformamide, dimethyl sulfoxide, and formamide, respectively.

While certain representative embodiments and details have been shown to illustrate the present invention, it will be apparent to those skilled in this art that various changes and modifications can be made and that, within the scope of the appended claims, the invention may be practiced otherwise than as specifically described and claimed.

### References:

1. Dorozhkin SV. Calcium orthophosphates and human beings: a historical perspective from the 1770s until 1940. Biomatter, 2012;2(2):53-70. doi:10.4161/biom.21340
2. Habraken W, Habibovic P, Epple M, et al. Calcium phosphates in biomedical applications: materials for the future?. Materials Today, 2016, 19(2): 69-87.3.1
3. Canillas M, 3. Pena P, Antonio H, et al. Calcium phosphates for biomedical applications. Boletin de la Sociedad Española de Cerámica y Vidrio, 2017, 56(3): 91-112.
4. Combes C, Rey C. Amorphous calcium phosphates: synthesis, properties and uses in biomaterials. Acta Biomaterialia, 2010, 6(9): 3362-3378.
5. Daculsi G, Laboux O, Malard O, et al. Current state of the art of biphasic calcium phosphate bioceramics. Journal of Materials Science: Materials in Medicine, 2003, 14(3): 195-200.
6. Komath M, Varma H K. Development of a fully injectable calcium phosphate cement for orthopedic and dental applications. Bulletin of Materials science, 2003, 26(4): 415-422.
7. Suzuki O, Imaizumi H, Kamakura S, et al. Bone regeneration by synthetic octacalcium phosphate and its role in biological mineralization. Current Medicinal Chemistry, 2008, 15(3): 305-313.
8. Ahn E S, Gleason N J, Nakahira A, et al. Nanostructure processing of hydroxyapatite-based bioceramics. Nano Letters, 2001, 1(3): 149-153.
9. Schmitz J P, Hollinger J O, Milam S B. Reconstruction of bone using calcium phosphate bone cements: a critical review. Journal of Oral and Maxillofacial Surgery, 1999, 57(9): 1122-1126.
10. https://www.nature.com/articles/s41467-020-15333-6
11. https://doi.org/10.1016/j.saa.2019.117892
12. https://doi.org/10.1016/S0013-4686(02)00812-5
13. https://www.ias.ac.in/article/fulltext/seca/068/03/0109-0122
14. https://www.journal.csj.jp/doi/pdf/10.1246/bcsj.44.316
15. https://doi.org/10.1016/j.saa.2017.01.062
16. https://doi.org/10.1063/1.5129464
17. Nat. Commun. 11, 1546, 2020; https://doi.org/10.1038/s41467-020-15333-6
18. Cichocka, M. O., Angstrom, J., Wang, B., Zou, X. D. & Smeets, S. High through-put continuous rotation electron diffraction data acquisition via software automation. J. Appl. Crystallogr. 51, 1652-1661 (2018)
19. Sheldrick, G. SHELXT-Integrated space-group and crystal-structure determination. Acta Crystallogr. Sect. A 71, 3-8 (2015)
20. Sheldrick, G. A short history of SHELX. Acta Crystallogr. Sect. A 64, 112-122 (2008)
21. Burla, M. C. et al. Crystal structure determination and refinement via SIR2014. J. Appl. Crystallogr. 48, 306-309 (2015)
22. https://www.nature.com/articles/s41467-020-15333-6#Sec6

## Claims

1. A hybrid-layered dicalcium phosphate (HLCP), wherein the HLCP is crystalline and comprises, or consists essentially of, or consists of alternating inorganic layers of dicalcium phosphate units and organic layers of molecules of at least one organic solvent or at least one organic active substance or a mixture of both.

2. The HLCP according to claim 1, wherein the molecules of the at least one organic solvent are selected from the group consisting of methanol, formamide, dimethyl sulfoxide, dimethylformamide, glycerol, ethylene glycol, ethanol, and mixtures thereof.

3. The HLCP according to claims 1 or 2, wherein the HLCP is crystalline dicalcium phosphate methanol (CaHPO₄ • CH₃OH), crystalline dicalcium phosphate formamide (Ca-HPO₄ • HCONH₂), crystalline dicalcium phosphate dimethyl sulfoxide (CaHPO₄ • (CH₃)₂SO), crystalline dicalcium phosphate dimethylformamide (CaHPO₄ • HCON(CH₃)₂), crystalline dicalcium phosphate glycerol (CaHPO₄ • HO-CH₂-CH(OH)-CH₂-OH), crystalline dicalcium phosphate ethylene glycol (CaHPO₄ • HO-CH₂-CH₂-OH), or crystalline dicalcium phosphate ethanol (CaHPO₄ • C₂H₅OH).

4. The HLCP according to claim 3, wherein the dicalcium phosphate methanol crystals have a monoclinic unit cell with space group P2₁/c and unit cell parameters a = 1.0153 nm, b = 0.6814 nm, c = 0.7725 nm, α = γ = 90°, β = 106.001; the dicalcium phosphate formamide crystals have a monoclinic unit cell with space group P2₁/c and unit cell parameters a = 1.0470 nm, b = 0.6784 nm, c = 0.7879 nm, α = γ = 90°, β = 105.578°; the dicalcium phosphate dimethyl sulfoxide crystals have a monoclinic unit cell with space group P2₁/c and unit cell parameters a = 1.12442 nm, b = 0.6759 nm, c = 0.7803 nm, α = γ = 90°, β = 106.065°; or the dicalcium phosphate dimethylformamide crystals have a monoclinic unit cell with space group P2₁/c and unit cell parameters a = 1.209 nm, b = 0.6774 nm, c = 0.782 nm, α = γ = 90°, β = 107.24°.

5. The HLCP according to any one of claims 1 to 4, wherein the at least one organic active substance is selected from the group consisting of antibiotics, antiphlogistics, drugs for the treatment of bone or dental diseases, drugs to promote regeneration of bone or dental tissue, and drugs to prevent rejection of bone or dental implants.

6. The HLCP according to any one of claims 1 to 5 for use in bone tissue engineering or regenerative medicine, as or in bioceramics, as or in biocomposites, as or in bone scaffolds, as or in implants, as or in implant coatings, as or in CaP printable powders or liquids or as additives, and/or as an active substance carrier.

7. The HLCP according to anyone of claims 2 to 5, wherein the at least one organic solvent molecule is dimethyl sulfoxide for use as a medicament with analgesic, antiphlogistic, vasodilatory, and anti-edematous effects.

8. Use of an HLCP according to any one of claims 1 to 5 in bone tissue engineering or regenerative medicine as or in bioceramics, as or in biocomposites, as or in bone scaffolds, as or in implants, as or in implant coatings, as or in CaP printable powders or liquids or as additives and/or as an active substance carrier.

9. A method for preventing or reducing bacterial infections, preventing or reducing inflammations, treating bone or dental diseases, promoting the regeneration of bone or dental tissue, and/or preventing rejection of bone or dental implants comprising the step of:
implanting a material comprising an HLCP according to any one of claims 1 to 5 carrying at least one organic active substance selected from the group consisting of antibiotics, antiphlogistics, drugs for the treatment of bone or dental diseases, drugs to promote regeneration of bone or dental tissue, and drugs to prevent rejection of bone or dental implants.

10. A method for treating pain, inflammations, hypertension, and/or edema comprising the step of:
implanting a material comprising an HLCP according to any one of claims 2 to 5, wherein the HLCP comprises dimethyl sulfoxide as the at least one organic solvent molecule.

11. A method for the manufacture of an HLCP (according to any one of claims 1 to 5) comprising the step of:
incubating a crystalline or precipitate calcium phosphate material with at least one organic solvent or at least one organic active substance or a mixture of both comprising 0 to 15 vol.% of water.

12. The method of claim 11, wherein the calcium phosphate material is selected from the group consisting of amorphous dicalcium phosphate containing 0-2 moles of water per mole of dicalcium phosphate, crystalline dicalcium phosphate monohydrate, and dicalcium phosphate dihydrate.

13. The method of claims 11 or 12, wherein the at least one organic solvent is selected from the group consisting of methanol, formamide, dimethyl sulfoxide, dimethylformamide, glycerol, ethylene glycol, and ethanol.

14. The method according to any one of claims 11 to 13, wherein
(i) for the manufacture of dicalcium phosphate methanol (CaHPO₄ • CH₃OH) a calcium phosphate material selected from the group consisting of amorphous dicalcium phosphate containing 0-2 moles of water per mole of dicalcium phosphate, crystalline dicalcium phosphate monohydrate, and dicalcium phosphate dihydrate is incubated with methanol as an organic solvent, wherein the methanol comprises 0 to 15 vol.% water,
(ii) for the manufacture of dicalcium phosphate formamide (CaHPO₄ • HCONH₂) a calcium phosphate material selected from the group consisting of amorphous dicalcium phosphate containing 0-2 moles of water per mole of dicalcium phosphate, crystalline dicalcium phosphate monohydrate, and dicalcium phosphate dihydrate is incubated with formamide as an organic solvent, wherein the formamide comprises 0 to 15 vol.% water,
(iii) for the manufacture of dicalcium phosphate dimethyl sulfoxide (CaHPO₄ • (CH₃)₂SO) a calcium phosphate material selected from the group consisting of amorphous dicalcium phosphate containing 0-2 moles of water per mole of dicalcium phosphate, crystalline dicalcium phosphate monohydrate, and dicalcium phosphate dihydrate is incubated with dimethyl sulfoxide as an organic solvent, wherein the dimethyl sulfoxide comprises 0 to 15 vol.% water,
(iv) for the manufacture of dicalcium phosphate dimethylformamide (CaHPO₄ • HCON(CH₃)₂) a calcium phosphate material selected from the group consisting of amorphous dicalcium phosphate containing 0-2 moles of water per mole of dicalcium phosphate, crystalline dicalcium phosphate monohydrate, and dicalcium phosphate dihydrate is incubated with dimethylformamide as an organic solvent, wherein the dimethylformamide comprises 0 to 15 vol.% water,
(v) for the manufacture of dicalcium phosphate glycerol (CaHPO₄ • HO-CH₂-CH(OH)-CH₂-OH) a calcium phosphate material selected from the group consisting of amorphous dicalcium phosphate containing 0-2 moles of water per mole of dicalcium phosphate, crystalline dicalcium phosphate monohydrate, and dicalcium phosphate dihydrate is incubated with glycerol as an organic solvent, wherein the glycerol comprises 0 to 15 vol.% water,
(vi) for the manufacture of dicalcium phosphate ethylene glycol (CaHPO₄ • HO-CH₂-CH₂-OH) a calcium phosphate material selected from the group consisting of amorphous dicalcium phosphate containing 0-2 moles of water per mole of dicalcium phosphate, crystalline dicalcium phosphate monohydrate, and dicalcium phosphate dihydrate is incubated with ethylene glycol as an organic solvent, wherein the ethylene glycol comprises 0 to 15 vol.% water, or
(vii) for the manufacture of dicalcium phosphate ethanol (CaHPO₄ • C₂H₅OH) a calcium phosphate material selected from the group consisting of amorphous dicalcium phosphate containing 0-2 moles of water per mole of dicalcium phosphate, crystalline dicalcium phosphate monohydrate, and dicalcium phosphate dihydrate is incubated with ethanol as an organic solvent, wherein the ethanol comprises 0 to 15 vol.% water.

15. A method for the conversion of one HLCP into another HLCP, wherein an HLCP according to any one of claims 1 to 5 is incubated with a different organic solvent.
